# EUROPEAN PATENT SPECIFICATION

(11) **EP 0 534 887 B1**
(45) Date of publication and mention of the grant of the patent: **09.10.1996**
(21) Application number: 92500084.6
(22) Date of filing: 02.07.1992
(51) Int. Cl.: A01N 37/20

(54) **Germicidal compositions containing iodine compounds**
Iod-Verbindungen enthaltende germizide Zusammensetzungen
Compositions germicides contenant des composés d'iode

(30) Priority: 03.07.1991 ES 9101563
(43) Date of publication of application: 31.03.1993
(73) Proprietor: Garcia Nunez, Maria Rosalia, E-28924 Alcorcon (Madrid) (ES)
(72) Inventor: Garcia Nunez, Maria Rosalia, E-28924 Alcorcon (Madrid) (ES)
(74) Representative: Alonso Langle, Emilio

(56) References cited:
- EP-A- 0 252 310
- WO-A-91/15120
- AU-A- 333 976
- FR-A- 2 379 508
- US-A- 4 042 368

## Description

The invention refers to a process for the preparation of iodine derivatives with germicidal activity. In addition, the invention provides new germicidal compositions which comprise the iodine derivatives of the invention together with other chemical products. These products have different and specific functions in order to carry out a sterilizing and disinfecting activity or to synergize and improve the main efect of the active iodine compounds.

The iodine compounds provided by the invention have a complex structure and include some high molecular weight molecules where elemental iodine has been fixed by means of a shifting reaction.

In particular, the iodine compounds provided by the invention correspond to the following formulae:
a) dimethyl alkyl benzalconium iodide (compound I) having the following general formula : 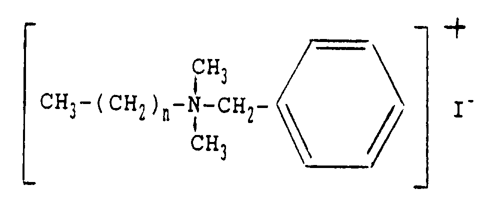 where n is a whole number between 1 and 20 (both included) and
b) quaternary ammonium amide iodide (compound II) having the following general formula : 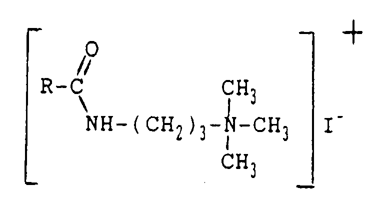 where R is a decylenic group.

### USE OF THE INVENTION

The iodine compounds I and II provided by the invention are suitable for preventing hospital infection, their main activity being to avoid and to control said infection which frequently arises during hospital practice, for example as post-surgical complication or by any other means, thus implying a reduction in the death rates for these causes and a reduction in the hospital costs.

In addition compounds I and II of the present invention may be used in the industry, in particular in the food canning and in the pharmaceutical industry.

### BACKGROUND ART

### 1.Principles

Hospital infection can be transmitted by different ways and can affect any person in an hospital. Not only patients, but also the hospital personnel and even the visitors may be affected by the infection. The problem is hard to solve and very important. According to Mr. William Foege, 9% of all the American patients who have to stay in hospitals are infected by hospital infections not directly related to the original cause for going to the hospital. The increase in sanitary costs is also very relevant.

The tests at hospital scale carried out with the germicidal compositions provided by the invention incorporating the iodine compounds I and II previously mentioned lead to results which allow to state that the problem of infection can be satisfactorily solved by means of said germicidal composition.

No references have been found to other similar systems or compounds which could solve not only hospital infection, but infections caused by germs which negatively affect the industry provoking economical losses. In the searched literature only few references have been found relating to some compounds which at the allowable use concentrations do not solve the problem satisfactorily.

### 2. State of the art

For a better understanding of the present invention the systems and agents presently used against microorganisms will be shortly referred.

It is known that sterilization consists in the total destruction of all microorganisms even in spore form, while, in general, disinfection refers to the destruction of microorganisms which cause infectious diseases.

The systems used for sterilization can be physical or chemical. Among the physical systems, the following could be mentioned:
a) Filtration suitable only for liquids and air.
b) Dry heat e.g. burning or flaming.
c) Wet heat at normal pressure like pasteurization and tyndalization.
d) Wet heat at high pressure, using pressure cooker.
e) Ultrasounds, specially suitable for bacteria.
f) Radiation, in particular ultraviolet,ionizing, gamma and radiosterilization. Ultraviolet radiation is not very effective against certain viruses and other Gram-negative germs. Ionizing radiations have a partially bactericidal effect and a dubious effectiveness when sterilizing plastic materials. X and gamma radiations are letal for almost all type of germs but cannot be used in hospitals or in industrial processes. Radiosterilization has the disadvantage that it requires cobalt 60 generators which are not provided in all the communities.
g) The use of gases like i) ethylene oxide which has the disadvantage of generating toxic residues which have to be aerated for disposal and, in addition, it produces an explosive mixture. Therefore, it has been forbidden in the CE, ii) beta propyol lactone which is not usable for being carcinogenic and iii) cyanhydric acid, very limited for its toxicity.

The chemical products or agents normally used for the destruction of microorganisms must have the following features:
a) strong germicidal power and broad spectrum.
b) solubility in polar and non polar environments.
c) low surface tension.
d) not toxic, not corrosive and preferably odourless.
e) stability and compatibility with other chemical substances used in cleaning and disinfection (soap, etc).

Among the chemical products normally used in disinfection, the following should be mentioned:
a) Oxidants for example i) hydrogen peroxide of very brief action; ii) potassium permanganate, not very much used because it stains the treated surfaces; and halides which will be reviewed later on.
b) Acids, not very much used for their corrosive effect.
c) Alkaline substances, like sodium hydroxide, which is toxic at the use dosage.
d) Alcohols, like ethanol, which is not germicidal but synergizes the effect of iodine. It can also decrease the germicidal activity of other agents like phenol, formaldehide and heavy metals.
e) Aldehydes, for example i) formaldehyde, forbidden by the EC for being carcinogenic; ii) formol which is not very much used because of its toxicity to humans and because it produces toxic gases; iii) glutaldehyde, not stable and toxic.
f) Phenolic derivatives, for example i) phenol, which is irritant to the skin; ii) hexachlorphenol, which is only bacteriostatic and in addition produces diseases in the central nervous system of new born babies which have been bathed with 3% solution of this substance; iii) cresol, which are not used because of its toxicity and iv) picric acid which is also very limited because of its toxicity.
g) Halides for example i)chlorine and certain derivatives like sodium or calcium hypochlorite and the chloramines and ii)iodine which is a good disinfectant and is used normally as iodine dye. Nevertheless, iodine derivatives have in general a certain irritant effect on the skin and for this reason they are normally mixed with non ionic surfactants which decrease said irritant effect thus resulting iodine compounds like iodine polyvinylpyrrolidone which has a very strong bactericidal effect but cannot be used because it has a very low water solubility where it splits liberating iodine.
h) Diguanidine compounds, e.g. chlorhexidine where the active principle is bichlorphenyl-diguanidohexane which is a good bactericide in particular against Gram-positive microorganisms, but stains the treated textiles and is not compatible with formaldehide, anionic compounds, soaps etc. thus limiting its use even more.
i) Surfactants which are used in 0.5% alcoholic solution for washing hands and skin and in the food industry. Among this group the humectants which can be cationic, anionic or non ionic should be highlighted. Benzalconium chloride has been used very much but it has been stated that its effect is decreased by proteins and cellulose fibers.
j) Metalic compounds, for example i) mercury compounds (soluble and not soluble) some of them having toxic and irritant effects; ii) silver compounds, not very effective for disinfecting large areas because of its limited germicidal activity; iii) cupric salts usable as fungicides in open containers but requiring a constant degree of humidity, thus limiting its use;
k) Organometalic compounds like mertiolate and mercury-chrome, effective for disinfection of sores, but being affected by light, its use at the industrial scale is therefore discarded.
l) Other chemical agents used as disinfectants are acrydine dyes and pine essential oils which, in some cases, can be potentially toxic (acrydine dyes) and are frequently used for disinfecting and deodorizing closed rooms.

As it can be appreciated none of the physical or chemical systems presently used in disinfection or sterilization properly solve the problems arising during disinfection processes and many of them present , in addition, limitations to their use. However, by using the germicidal compositions of the present invention which include, among other substances, the iodine derivatives I and II previously indicated above it is possible to solve the mentioned problems and to properly disinfect all type of rooms and equipments.

### DETAILED DESCRIPTION OF THE INVENTION

According to one embodiment of the present invention, a process for the obtention of iodine derivatives I and II is provided, said iodine derivatives being
a) dimethyl alkyl benzalconium iodide (derivative I) having the following general formula: 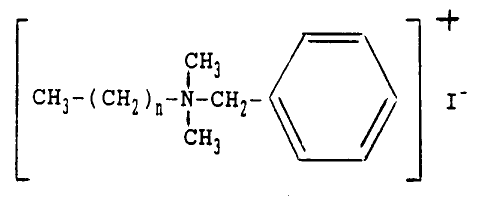 where n is a whole number between 1 and 20 (both included) and
b) quaternary ammonium amide iodide (derivative II) having the following general formula: 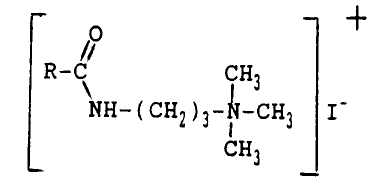 where R is a decylenic group.

A decylenic group is an alkenyl group having 10 carbon atoms and with a double bond in any position of the group.

Compounds I and II mentioned above could be obtained from the corresponding chlorides by reaction with elemental iodine thereby causing a shifting of chlorine by iodine. A more detailed description of this process will be given when reviewing the obtention procedure of the germicide compositions of the present invention. According to another embodiment of the present invention germicide compositions suitable for sterilizing and disinfecting rooms, equipments and apparatus are provided, said compositions including iodine compounds I and II as well as other chemical substances which have the previously mentioned activity. The chemical substances which can be present in the compositions together with the iodine compounds I and II are the following:
- an oxyethylenated fatty alcohol with 8 mols of ethylene oxide,
- resublimated elemental iodine.
- glycerol in order to avoid the astringent action of eventual free iodine in the composition;
- a monoglyceride of polyoxyethylated fatty acid in order to decrease the surface tension of the environment, thereby achieving a rapid humection of the cell walls in the case of bacteria or breaking the different proteins which form the virus capsule;
- glycol in order to avoid that the free iodine reacts with other components of the germicidal composition.

In a preferred embodiment of the present invention a germicidal composition is provided, constituted by:

| | |
|---|---|
| - dimethyl-stearyl benzalconium (I) iodide | 45% |
| - N-1-(3-trimethylammonium)-propyl-undecylamid (II) iodide | 10% |
| - oxyethylated fatty alcohol with 8 mols E.O. | 10% |
| - resublimated metalic iodine | 4% |
| - monoglyceride of a polyoxyethylated fatty acid | 10% |
| - glycerol | 5% |
| - glycol | 16% |

All the proportions are indicated in weight percentage referred to the total weight of the composition.

The germicidal compositions of the present invention may be obtained by a process comprising the following steps:
a) loading a reactor provided with stirring means with the appropriate amounts of a previously melted dimethylalkylbenzalconium chloride;
b) adding while smoothly stirring the appropriate amount of the quaternary ammonium amide of undecylenic acid;
c) adding the appropriate amount of glycol.
d) slowly adding resublimated elemental iodine in the appropriate stoichiometric proportion while increasing the stirring;
e) adding the appropriate amounts of:
   i) monoglyceride of polyoxyethylated fatty acid;
   ii) glycerol;
   iii) oxyethylenated fatty alcohol with 8 mols ethyle ne oxide;
f) adding mineral acid until a pH of apprrox. 4.5 is obtained.

In general, the process is carried out at a temperature slightly over 20ºC, the stirring speed being in the range between 400 and 1600 rpm.

By this way of operation, the shifting of the chloride present in the reacting substances and its substitution by iodine is achieved, the above mentioned iodine derivatives I and II being formed thereby.

It has been stated that the germicidal compositions-provided by the present invention perform their activity by the following mechanism:
- extraction of essential cellular metabolites;
- denaturalization of cellular proteins;
- reaction with cellular lipids;
- provoking an enzymatic desequilibrium and inhibiting essential coenzymes for the microorganisms and
- expulsing the vital matter out of the cell through the membrane by disrupting the osmotic balance.

This mechanism enables the germicidal compositions provided by the present invention to be very effective, not only before Gram-positive and Gram-negative bacteria, but against viruses as well and therefore they can be used for sterilizing and disinfecting rooms, equipment, apparatus etc....

It has been stated that the germicidal compositions provided by the present invention, in particular the previously mentioned preferred composition is not irritant, non sensitizing and non mutagenic at the use dosages tested. This is illustrated by examples 5 to 7. The composition is very effective against different types of germs (example 2).

The skilled man will, in view of the present description, notice that the invention resides in the shifting and substitution of chloride by iodine in a quaternary ammonium salt, the germicidal and fungicidal activity of the iodine molecules of the invention being provided by this change.

All the other possible modifications like the substitution of the benzyl group of compound I by other aromatic groups as well as variations in the number of -CH₂- groups or their arrangement (lineal or branched) are secondary changes which do not affect the germicidal or fungicidal activity of compounds I and II of the present invention.

Following, some examples of different ways of carrying the process of the invention are given, as well as some other examples illustrating the germicidal activity of the considered compositions.

### EXAMPLE 1

A stainless steel reactor provided with stirring means is loaded with 450g of previously melted dimethyl-stearyl benzalconium chloride. Stirring is then started at a speed of 500 rpm and 100g of undecylenic acid amide are then added at a temperature over 20ºC. Stirring is maintained until a complete mixture of all the components is achieved, approximately after 10 minutes. Later 160 g of glycol are added while maintaining the same stirring and temperature conditions until complete mixing. 40 g of resublimated elemental iodine are then added very slowly while the stirring speed is increased up to 1500 rpm. Stirring is maintained until iodine is completely dissolved and has reacted with the other components. The final mixture should not have suspended particles.

The following compounds are then added to the obtained mixture:
i) 100 g of oxyethylenated fatty alcohol with 8 mols of ethylene oxide.
ii) 50 g of glycerol and
iii) 100g of the monoglyceride of the polyoxyethylenated fatty acid;
stirring is maintained at least during 15 minutes and finally a mineral acid is added until the resultant solution reaches a pH of 4.5 approx. Stirring is maintained for one hour. After stating that no residues of free iodine are present, the obtained composition can be bottled.

### EXAMPLE 2

### Test of efectiveness against different types of germs

Some microbiological tests were carried out for determining the microbicidal activity of the composition obtained according to Example 1. Three different types of microorganisms were tested: bacteria, yeast and fungi.

A bouillon made of meat and glucose yeast was used as culture medium and the germs were:
i) Staphylococcus aureus;
ii) Saccharomyces ellipsoideus; and
iii) Penicilium crysogenum.

The consecutive dilution technique was used: 1, 5, 10, 20, 50, 100 and 200 ppm. 48 hours cultures of the germs indicated above were inoculated (0.01 cm³ in each case). The growth measurement was made by nefelometry.

In a second set of tests different dilutions were used interpolating the doses between those of the first experiment were growth was first detected. Finally, the germicide activity was tested by viability culture in agar environment. The following results were obtained:

| **FIRST EXPERIMENT** | |
|---|---|
| Germ | Germicide Activity (no growth) Dose comprised between (ppm) |
| Staphylococcus aureus | 1 and 5 |
| Saccharomyces ellipsoideus | 10 and 20 |
| Penicilium crysogenum | 20 and 30 |

| **SECOND EXPERIMENT** | | |
|---|---|---|
| Germ | Microbicide activity (ppm) | One part in |
| Staphylococcus aureus | 3 | 333.333 |
| Saccharomyces ellipsoideus | 12 | 85.333 |
| Penicilium crysogenum | 22 | 45.000 |

These results indicate that microbicidal effect of the germicidal composition of Example 1 against the tested germs.

### EXAMPLE 3

### Test of antimicrobial activity in drinking water

A test was carried out for searching the antimicrobial activity of the composition obtained according to Example 1 in relation to microorganisms contained in drinking water. The test was carried out according to the swiss norm Clinical Microbiology ASM 1991 which briefly comprises:
a) diluting the germicidal composition in sterile deionized water.
b) homogenizing 10 ml of drinking water sample and 10 ml of the diluted germicidal composition in a vibrating device.
c) maintaining the mixture in contact during 15 minutes and transferring 1 ml of said sample to 10 ml of Müller-Hinton gel heated at 45ºC and inmediately transferring it to a culture plate. Simultaneously 1 ml of the mixture water-germicidal composition is transferred to 1 ml of double concentred Müller-Hinton gel. The obtained preparations were incubated at 37ºC for 24 hours.

Following results were obtained:

### 1. Microbiological test of the drinking water used

1.1 Number of germs per ml : 407.5
1.2 Isolated germs:
   Pseudomonas putida
   Xanthomonas maltophilia
   Streptococcus
   Chromobacter
   Staphylococcus coagulase
   Bacillus sp
   Aspergillus niger
   Aspergillus sp

### 2. Antimicrobial power

| Dilutions of the germicide composition | | | | | | | |
|---|---|---|---|---|---|---|---|
| | 10⁻² | 5x10⁻² | 10-3 | 2.5x10⁻³ | 5x10⁻³ | 10-4 | 1.5x10⁻⁴ |
| Growth | - | - | - | - | - | + | + |

From the obtained results it can be stated that the germicidal composition can be used for decontaminating drinking water at a dilution comprised between 1/2500 and 1/5000.

### EXAMPLE 4

### Oral acute toxicity test

Some tests were carried out in order to obtain information about potential health hazards as a result of exposures to single doses of the germicidal composition of example 1.

Wistar albino rats with a weight comprised between 400 and 550 g were selected before starting the experiment, which was carried out according to the Comunity norms (Directive 79/831, Annex VIII part B, B2 (Toxicological methods for the evaluation of chemical substances). The experiment lasted 7 days and afterwards the surviving animals were killed.

Two different dilutions of the germicidal composition of example 1 were used: 1/10 and 1/30. The single dose was 2000 microliter/kg weight and was applied on the first day of the experience. Mortality, morbidity, behaviour and signs of toxicity after the application of the dose were observed after 30 minutes, 4 hours, 24 hours, 48 hours until 7 days. The obtained results were:

| **TEST A** Number of tested animals: 8 (male) Dose: 2000 microliter/kg weight Dilution of the germicide composition: 1/10 | | | |
|---|---|---|---|
| In. weight (g) | Dose(ul) | Final weight(g) | Observations |
| 502 | 1000 | 473 | Death |
| 489 | 980 | 395 | Death |
| 429 | 860 | 390 | Death |
| 434 | 870 | 428 | Death |
| 423 | 850 | 401 | Death |
| 460 | 920 | 284 | Death |
| 443 | 890 | 419 | Death |
| 455 | 910 | 455 | Death |

All the animals died before finishing the test. Therefore, at 1/10 dilution the germicide composition has a toxic lethal performance on test animals.

| **TEST B** Number of animals tested: 8 (male) Dose: 2000 microliter/kg weight Dilution of the germicidal composition: 1/30 | | | |
|---|---|---|---|
| In. weight (g) | Dose(ml) | Final weight(g) | Observations |
| 414 | 0.8 | 380 | Dyspnoea |
| 506 | 1.0 | 400 | Dyspnoea |
| 532 | 1.1 | 471 | Dyspnoea |
| 493 | 1.0 | 474 | Dyspnoea* |
| 546 | 1.1 | 513 | Dyspnoea |
| 499 | 1.0 | 458 | Slight dyspnoea |
| 461 | 0.9 | 397 | Slight dyspnoea |
| 473 | 0.9 | 438 | Moderate dyspnoea |

| | | | |
|---|---|---|---|
| * Death one day after application of the dose | | | |

From this experiment, it can be inferred that the DL₅₀ must lie between 110 mg/kg (dilution 1/10) and 36.6 mg/kg (dilution 1/30).

### EXAMPLE 5

### Test of retarded hipersensibility in Guinea pigs

Some tests were carried out in order to obtain information about potential health hazards, in particular skin sensitization as a result to the exposure to single doses of the germicide compositions of example 1.

For the experiment albino Guinea pigs 7-8 weeks old and with a weight comprised between 450 and 700 g were shaved on the places where the samples were going to be applied. The experiment was performed according to the EC norms already mentioned in the present description during a period of 28 days.

The sample diluted 1/300 was applied on the flank of the animal in the form of a non porous dressing. The animals were separated in two groups: one control group formed by 5 animals and other group constituted by 5 males and 5 females.

### Operating procedure:

a) Induction step
24 hours before the treatment as well as on the 7th and 14 th day the animals were shaved on their flanks and on the 1st, 8th and 15th days the composition was applied at a dose of 0.5 ml. The control animals passed the same treatment but without applying the germicidal composition to them.
b) Final step
On the 28th day, a skin area was shaved on all the animals (trated and control). On the 28th day, all the animals were treated by application of 0.5 ml during 6 hours. Afterwards, the dressing and the skin were gently separated. The following results were obtained:

| CONTROL GROUP Dilution: 1/300 | | | | | | | |
|---|---|---|---|---|---|---|---|
| SEX | In. W. | Fin. W. | Days | | | | |
| | | | 0 | 7 | 14 | 28 | 29-30 |
| M | 582 | 603 | - | - | - | 0 | 0 |
| M | 643 | 660 | - | - | - | 0 | 0 |
| M | 660 | 708 | - | - | - | 0 | 0 |
| M | 569 | 595 | - | - | - | 0 | 0 |
| M | 590 | 613 | - | - | - | 0 | 0 |

| EXPERIMENTALL GROUP Dilution: 1/300 | | | | | | | |
|---|---|---|---|---|---|---|---|
| SEX | In. W. | Fin. W. | Days | | | | |
| | | | 0 | 7 | 14 | 28 | 29-30 |
| M | 609 | 652 | 0 | 0 | 0 | 0 | 0 |
| M | 591 | 626 | 0 | 0 | 0 | 0 | 0 |
| M | 646 | 677 | 0 | 0 | 0 | 0 | 0 |
| M | 626 | 649 | 0 | 0 | 0 | 0 | 0 |
| M | 515 | 559 | 0 | 0 | 0 | 0 | 0 |
| M | 545 | 567 | 0 | 0 | 0 | 0 | 0 |
| M | 539 | 558 | 0 | 0 | 0 | 0 | 0 |
| M | 547 | 564 | 0 | 0 | 0 | 0 | 0 |
| M | 595 | 619 | 0 | 0 | 0 | 0 | 0 |
| M | 496 | 523 | 0 | 0 | 0 | 0 | 0 |
| Evaluiation of skin reaction: Value 0: No irritation; no reaction Value -: Slight irritation; | | | | | | | |

In view of these results, it can be inferred that on the tested conditions the composition of example 1 does not cause any retarded hypersensibility by contact.

The same experiment was carried out with a 1/10 dilution, always obtaining no evidence of irritation.

### EXAMPLE 6

### Irritation/corrosion test on rabbits

A sample (0,5 g diluted 1/10) of the composition obtained according to example 1 was applied on 3 albino rabbits by means of an occlusive dressing on a 6 cm² surface. The adjacent non treated skin portions constituted the control for the test. Observations were carried out 1, 24, 48 and 72 hours after application and also after 7 days in order to control retarded reactions.

After the 7th day no irritation was noticed on any animal. Therefore, it can be inferred that the test dose is not irritant.

### EXAMPLE 7

### Mutagenic reversion test

In order to search if the germicide composition according to example 1 was carcinogenic or mutagenic, the Ames and Purchase tests were performed.

A 100 microliter sample diluted 1/50000 of the composition of example 1 was used. The obtained results permit the conclusion that said germicidal composition in the tested conditions does not provoke mutations in the bacteria. Thus, under the tested conditions, the germicidal composition is not mutagenic.

## Claims

1. A broad spectrum germicidal composition comprising an effective amount of the following iodine compounds:
a) dimethyl alkyl benzalconium iodide having general formula I: 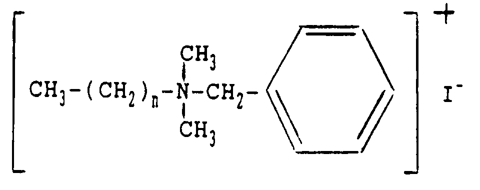 where n is a whole number between 1 and 20 (both included); and
b) quaternary ammonium amide iodide having general formula II: 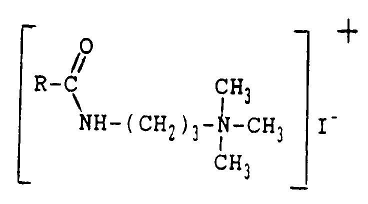 where R is a decylenic group.

2. Germicidal composition according to claim 1, characterized in that in addition to the compounds with formula I and II, it contains appropriate amounts of other chemical compounds which have a sterilizing and disinfecting function and which synergize and improve the activity of said compounds I an II.

3. A germicidal composition according to claim 2, characterized in that said additional compounds are selected among:
- an oxyethylenated fatty alcohol with 8 mols ethylene oxide;
- resublimated elemental iodine;
- glycerol;
- a monoglyceride of polyoxyethylanated fatty acid;
- glycol.

4. A germicidal composition according to the previous claims, characterized in that said composition comprises:
| | |
|---|---|
| - dimethyl-stearyl benzalconium (I) iodide | 45% |
| - N-1-(3-trimethylammonium)-propyl-undecylamide (II) iodide | 10% |
| - oxyethylated fatty alcohol with 8 mols E.O. | 10% |
| - resublimated elemental iodine | 4% |
| - monoglyceride of a polyoxyethylated fatty acid | 10% |
| - glycerol | 5% |
| - glycol | 16% |
All the proportions are indicated in weight percentage referred to the total weight of the composition.

5. Process for the obtention of a germicidal composition containing the iodine derivatives with general formulae I and II of claim 1, characterized in that it comprises the following steps:
a) loading a reactor provided with stirring means with the appropriate amount of a previously melted dimethylalkylbenzalconium chloride;
b) adding while smoothly stirring the appropriate amount of the quaternary ammonium amide of undecylenic acid;
c) adding the appropiate amount of glycol.
d) slowly adding resublimated metalic iodine in the appropriate stoichiometric proportion while increasing the stirring;
e) then adding the appropriate amounts of:
i) monoglyceride of polyoxyethylated fatty acid;
ii) glycerol;
iii) oxyethylated fatty alcohol with 8 mols ethylene oxide;
f) adding mineral acid until a pH of approx. 4.5 is obtained.

6. Use of the iodine compounds with general formulae I and II of claim 1 as germicides and fungicides.

7. Use of the germicidal compositions according to claims 1 to 4 for preventing infections in hospitals as well as in the food canning, pharmaceutical and other related industries.

8. Use of the composition according to claim 4 for preventing infections in hospitals and industries and for sterilizing rooms, equipments and apparatus.

## Patentansprüche

1. Eine Zusammensetzung mit einem umfangreichen keimtötenden Spektrum, die eine wirksame Menge folgender Jodverbindungen enthält:
a) Dimethyl-alkyl-benzalconium-jodid mit folgender allgemeiner Formel I: 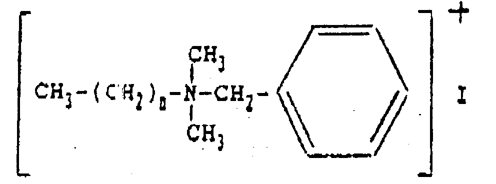 in der n eine ganze Zahl zwischen 1 und 20 (beide einschliesslich) ist, und
b) Amidjodid quaternären Amoniums mit folgender allgemeinen Formel II: 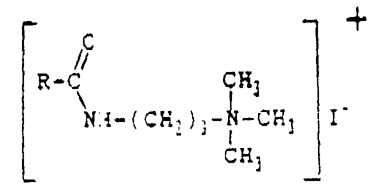 in der R eine Decylengruppe ist.

2. Eine keimtötende Zusammensetzung übereinstimmend mit Anspruch 1, dadurch gekennzeichnet, dass ausserdem die Verbindungen der Formeln I und II passende Mengen anderer chemischen Verbindungen enthalten, die eine sterilisierende und desinfizierende Funktion aufweisen und die Aktivität besagter Verbindungen I und II synergetizieren und verbessern.

3. Eine keimtötende Zusammensetzung übereinstimmend mit Anspruch 2, dadurch gekennzeichnet, dass besagte zusätzliche Verbindungen zwischen:
- einem oxyethylenisierten Fettalkohol mit 8 Mol Ethylenoxyd;
- Resublimiertem Elementarjod
- Glycerin
- einem Monoglycerid der polyoxyethylenisierten Fettsäure;
- Glykol
ausgewählt wird.

4. Eine keimtötende Zusammensetzung übereinstimmend mit den vorstehenden Ansprüchen, dadurch gekennzeichnet, dass diese Zusammensetzung folgendes enthält:
| | |
|---|---|
| - Dimethyl-Benzalconiumstearyl-Jodid | 45 % |
| - N-1-(3-trimethylamonium)-propyl-undecylamid (II)-Jodid | 10 % |
| - Oxyethylenisierter Fettalkohol mit 8 Mol Ethylenoxyd | 4 % |
| - Resublimiertes Elementarjod | 4 % |
| - Monoglycerid der polyoxyethylenisierten Fettsäure | 10 % |
| - Glycerin | 5 % |
| - Glykol | 16 % |
Alle Anteile werden als Gewichtsprozent bezogen auf das Gesamtgewicht der Zusammensetzung angegeben.

5. Ein Verfahren für die Herstellung einer keimtötenden Zusammensetzung, die Jodderivate der allgemeinen Formeln I und II des Anspruchs 1 enthält, dadurch gekennzeichnet, dass es folgende Schritte einschliesst:
a) das Beschicken eines mit Rührwerk ausgerüsteten Reaktors mit der passenden Menge eines vorher geschmolzenen Dimethylbenzalconium-Chlorids.
b) das Hinzufügen unter langsamem Rühren der passenden Menge eines quaternären Amoniumamids der Undecylsäure;
c) das Hinzufügen der passenden Menge Glykol;
d) das langsame Hinzufügen von resublimiertem Metalljod in einem stöchiometrisch passenden Anteil, während der Rührvorgang beschleunigt wird;
e) das darauffolgende Hinzfügen der passenden Mengen an:
i) Monoglycerid der polyoxyethylenisierten Fettsäure;
ii) Glycerin
iii) oxyethylenisierter Fettalkohol mit 8 Mol Ethylenoxyd
f) das Hinzufügen von Mineralsäure, bis ein pH von ca. 4,5 erreicht ist.

6. Verwendung der Jodverbindungen der allgemeinen Formeln I und II des Anspruchs 1 als Keimtöter und Fungizide.

7. Verwendung der keimtötenden Zusammensetzungen übereinstimmend mit den Ansprüchen 1 bis 4 für die Vorbeugung von Krankenhausinfektionen, sowie für Nahrungsmittel in Dosen, für die pharmazeutische Industrie und andere damit verbundene Industrien.

8. Verwendung der Zusammensetzung übereinstimmend mit Anspruch 4 für die Vorbeugung von Krankenhausinfektionen und für Industrien sowie für die Sterilisierung von Räumen, Ausrüstungen und Apparaten.

## Revendications

1. Une composition ayant un vaste spectre germicide qui contient une quantité effective des composés d'iode suivants:
a) iodure de dyméthyle alkyle benzalconium ayant la formule générale I: 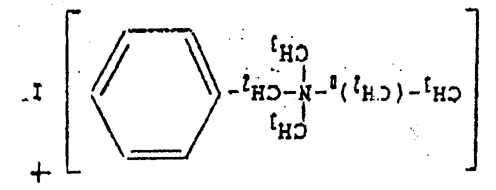 dans laquelle n est un nombre entier entre 1 et 20 (tous deux inclus); et
b) iodure d'amide d'ammonium quaternaire ayant la formule générale II: 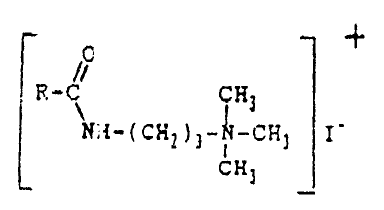 dans laquelle R est un groupe décilénique.

2. Une composition germicide conforme à la revendication I caractérisée par le fait qu'en plus des composés de formule I et II elle contient les quantités appropriées d'autres composés chimiques qui ont une fonction stérilisante et désinfectante qui synergisent et améliorent l'activité des composés I et II cités.

3. Une composition germicide conforme à la revendication 2 caractérisée par le fait que lesdits composés supplémentaires sont sélectionnés parmi:
- de l'alcool gras oxyethylénisé avec 8 moles d'oxyde d'éthylène.
- de l'iode élémentaire résublimé.
- de la glycérine.
- un monoglycéride d'acide gras polyoxyéthylénisé
- du glycole.

4. Une composition germicide conforme aux revendications antérieures caractérisée par le fait que ladite composition contient:
| | |
|---|---|
| - du iodure de dyméthyle stéaryle benzalconium | 45% |
| - du iodure de N-1-(3 triméthylamonium)-propylundécilamide (II) | 10% |
| - d l'alcool gras oxyéthylénisé avec 8 moles d'o.e. | 4% |
| - de l'iode élémentaire résublimé. | 4% |
| - du monoglycéride d'acide gras polyoxyéthylénisé | 10% |
| - de la glycérine | 5% |
| - du glycole. | 16% |
Toutes les proportions sont indiquées comme pourcentage du poids de référence par rapport au poids de la composition.

5. Un procédé pour l'obtention d'une composition germicide qui contient des dérivés d'iode ayant les formules générales I et II de la revendication 1, et dont la caractéristique est qu'il inclut les étapes suivantes:
a) la charge sur un réacteur équipé de moyens d'agitation de la quantité appropriée d'un chlorure de dyméthyl-benzalconium fondu au préalable.
b) l'addition, en agitant doucement, de la quantité appropriée d'une amide d'amonium quaternaire d'acide undécilénique.
c) l'addition de la quantité appropriée de glycole.
d) l'addition, avec douceur, d'iode métallique résublimé selon la proportion stoéchiométrique appropriée au fur et à mesure de l'augmentation de l'agitation.
e) puis l'addition des quantités appropriées de:
i) monoglycéride d'acide gras polyoxyéthylénisé;
ii) glycérine;
iii) alcool gras oxyéthylénisé avec 8 moles d'oxyde à'éthylène.
f) l'addition d'acide minéral jusqu'à l'obtention d'un pH d'environ 4,5.

6. Utilisation des composés d'iode ayant la formule générale I et II de la revendication 1 comme germicides et fongicides.

7. Utilisation des compositions germicides conformément aux revendications 1 à 4 pour la prévention d'infections dans les hôpitaux ainsi que dans les aliments en boîte dans les industries pharmaceutiques et autres en rapport avec ces dernières.

8. Utilisation de la composition conformément à la revendication 4 pour la prévention d'infections dans les hôpitaux et industries et pour la stérilisation des chambres, équipements et appareils.
